# EUROPEAN PATENT APPLICATION

(11) **EP 2 368 489 A1**
(43) Date of publication of application: **28.09.2011**
(21) Application number: 11152670.3
(22) Date of filing: 31.01.2011
(51) Int. Cl.: A61B 1/12, A61B 1/05

(54) **Imaging unit and endoscope**

(30) Priority: 26.03.2010 JP 2010073867
(71) Applicant: Fujifilm Corporation, Minato-ku Tokyo 106-0031 (JP)
(72) Inventor: Yamamoto, Goki, Kanagawa (JP); Takahashi, Kazuaki, Kanagawa (JP); Takasaki, Kosuke, Kanagawa (JP)
(74) Representative: HOFFMANN EITLE

(57) **Abstract**

An imaging unit (50) includes an objective optical system (35,36), an image sensor (59), a cover glass (71), a prism (55), and a heat conduction portion (81). The objective optical system (35,36) imports light of an image of a subject. The image sensor (59) forms an image of the light and outputs an imaging signal. The cover glass (71) is provided on an imaging surface of the image sensor (55) and seals the image sensor air-tightly. The prism (55) is disposed between the objective optical system (35,36) and the cover glass (71) so as to guide the light from the objective optical system (35,36) to the imaging surface. The heat conduction portion (81) touches the prism and extends from the prism towards the image sensor (59) so as to transmit heat of the image sensor (59) to the prism (55).

## Description

### BACKGROUND

### 1. Technical Field

The present invention relates to an imaging unit and an endoscope.

### 2. Related Art

In the medical field, endoscopes are used for medical diagnosis. An imaging unit having a solid-state image sensor such as a CCD image sensor is built in a front end of an insertion portion of an endoscope, which portion will be inserted into a body cavity. In the endoscope, a processing unit performs signal processing on an imaging signal obtained by the solid-state image sensor so that an image in the body cavity can be observed on a monitor.

The imaging unit of the endoscope is provided with the solid-state image sensor and an objective optical system for importing light incident thereon via an observation window provided in the front end of the insertion portion. A cover glass is disposed on an imaging surface of the solid-state image sensor with an air layer interposed therebetween. Further, in the imaging unit, a prism for guiding the incident light from the objective optical system to the imaging surface of the solid-state image sensor may be provided on the cover glass.

In the insertion portion of the endoscope, the solid-state image sensor or electronic components around the solid-state image sensor may be raised to higher temperature than the front end of the insertion portion due to heat generated by the driving. As a result, a difference in temperature occurs between an outer surface of the cover glass on the light entrance side and an inner surface of the cover glass on the solid-state image sensor side. When moisture is contained in a space where the solid-state image sensor is sealed with the cover glass, dew condensation may be formed on the inner surface of the cover glass. Particularly, water or air may be delivered to the front end of the insertion portion when the observation window is smeared, so that dew condensation is formed easily on the inner surface of the cover glass due to the temperature difference occurring between the front end of the insertion portion and the inside of the insertion portion. When dew condensation occurs on the inner surface of the cover glass, water drops are formed by the dew condensation so as to make it difficult to observe an image displayed on the monitor.

In the endoscope having the configuration in which a prism is provided between the objective optical system and the solid-state image sensor, the outer surface of the cover glass for sealing the solid-state image sensor abuts against the prism, The objective optical system including an objective lens etc. disposed on the front end side of the insertion portion of the endoscope is disposed on the light entrance side of the prism. Since the prism has a large heat capacity, it takes time till the outer surface of the cover glass abutting against the prism follows the temperature rise of the inner surface of the cover glass even when the temperature of the inner surface of the cover glass rises due to heat on the solid-state image sensor side. Therefore, a difference in temperature occurs easily between the outer surface of the cover glass located on the light entrance side and abutting against the prism and the inner surface of the cover glass located on the solid-state image sensing device side.

An endoscope with an imaging unit is disclosed in Japanese Patent No. 3004286.

Japanese Patent No. 3004286 discloses an endoscopic apparatus in which a cover lens of an imaging optical system is fixed to a front end portion of an insertion portion of an endoscope through a lens frame formed out of a material with high thermal conductivity, a solid-state image sensor is fixed by a thermally conductive fixing member insulated from the lens frame, and the fixing member and the lens frame are connected consecutively to each other through a thermally conductive member so that heat of the solid-state image sensor can be transmitted to the cover lens to prevent dew condensation on the cover lens.

The aforementioned document gives no description about suppression of dew condensation on a cover glass in the endoscope in which a prism is provided on a light entrance side of the cover glass for sealing the solid-state image sensor.

An object of the invention is to provide an imaging unit and an endoscope in which occurrence of dew condensation on a cover glass attached to an image sensor can be suppressed more surely.

### SUMMARY OF THE INVENTION

[1] According to an aspect of the invention, an imaging unit includes an objective optical system, an image sensor, a cover glass, a prism, and a heat conduction portion. The objective optical system imports light of an image of a subject. The image sensor forms an image of the light and outputs an imaging signal. The cover glass is provided on an imaging surface of the image sensor and seals the image sensor air-tightly. The prism is disposed between the objective optical system and the cover glass so as to guide the light from the objective optical system to the imaging surface. The heat conduction portion touches the prism and extends from the prism towards the image sensor so as to transmit heat of the image sensor to the prism.
[2] The imaging unit according to configuration [1] further comprising a circuit board which is mounted with the image sensor, wherein the heat conduction portion is disposed in contact with the circuit board.
[3] The imaging unit according to the configuration [1], wherein the heat conduction portion is disposed in contact with the image sensor.
[4] The imaging unit according to any one of the configuration [1] through the configuration [3], wherein the heat conduction portion is in surface-contact with a surface of the prism other than a light entrance surface and a light exit surface of the prism.
[5] The imaging unit according to any one of the configuration [1] through the configuration [4], further comprising:
   a heat insulation unit that is provided at least between the heat conduction portion and the cover glass so as to avoid direct contact therebetween.
[6] The imaging unit according to the configuration [5], wherein:
   the heat insulation unit is provided in an opposite surface of the heat conduction portion to a side where the heat conduction portion touches the prism.
[7] The imaging unit according to any one of the configuration [1] through the configuration [6], wherein the heat conduction portion is formed out of a thermally conductive resin.
[8] The imaging unit according to the configuration [7], wherein the thermally conductive resin includes at least one of an epoxy resin, an acrylic resin, a silicon resin and a urethane resin.
[9] The imaging unit according to the configuration [7] or the configuration [8], wherein the thermally conductive resin includes a thermally conductive filler.
[10] The imaging unit according to any one of the configuration [1] through the configuration [6], wherein the heat conduction portion is a thermally conductive sheet.
[11] The imaging unit according to the configuration [10], wherein the thermally conductive sheet is any one of a silicon sheet, a carbon nano sheet and a graphite sheet.
[12] An endoscope comprising:
   an insertion portion in which an imaging unit according to any one of the configuration [1] through [11] is built.

When the imaging unit is driven, temperature in and around the image sensor becomes higher than that in the objective optical system due to heat generated by the driving. Thus, heat is transmitted from the image sensor side to the inner surface of the cover glass on the image sensor side so as to increase the temperature in the inner surface of the cover glass. On the other hand, heat generated in and around the image sensor is transmitted to the prism through a heat conduction portion. Thus, a temperature difference between the temperature of the prism and the temperature of the image sensor side is narrowed. It is therefore possible to suppress occurrence of a temperature difference between the outer surface of the cover glass on the prism side and the inner surface of the cover glass on the image sensor side, and it is possible to suppress occurrence of dew condensation on the cover glass.

According to the invention, it is possible to provide an imaging unit and an endoscope in which occurrence of dew condensation on a cover glass attached to an image sensor is suppressed.

### BRIEF DESCRIPTION OF THE DRAWINGS

Fig. 1 is an overall configuration view of an endoscope.
Fig. 2 is a schematic external view showing a front end portion of an insertion portion of the endoscope.
Fig. 3 is a sectional view taken along the direction of arrows A-A in Fig. 2.
Fig. 4 is a sectional view taken along the direction of arrows IV-IV in Fig. 3.

### DETAILED DESCRIPTION

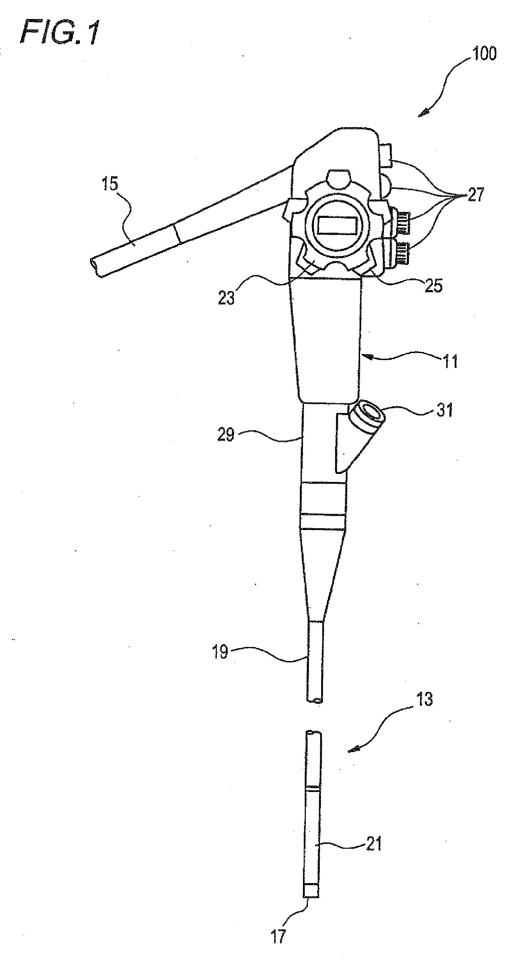

Fig. 1 is an overall configuration view of an endoscope.

An endoscope 100 has a body operation portion 11 and an endoscope insertion portion 13 which is provided consecutively to the body operation portion 11 and which will be inserted into a body cavity.

The body operation portion 11 has angle knobs 23 and 25, various buttons 27 such as an air/water-supply button, a suction button and a shutter button, a consecutive joint portion 29 extended toward the endoscope insertion portion 13 side, and a forceps insertion portion 31 provided in the consecutive joint portion 29. In addition, a universal cable 15 is connected to the body operation portion 11.

The endoscope insertion portion 13 has a soft portion 19, a curved portion 21 and a front end portion 17 which are provided in order in a direction from the body operation portion 11 side toward the front end.

The angle knobs 23 and 25 are rotated to serve for performing operation to drive and bend the curved portion 21 of the endoscope insertion portion 13.

The various buttons 27 are operated by a user to perform air/water supply, suction, imaging of a portion to be observed, etc. in the front end portion 17 of the endoscope insertion portion 13.

The forceps insertion portion 31 has an opening into which an operative instrument such as forceps will be inserted. The opening communicates with a forceps port formed in the front end portion 17 of the endoscope insertion portion 13. The forceps port will be described later.

A not-shown light guide connector is provided in a front end of the universal cable 15. The light guide connector is removably connected to a not-shown light source unit, by which illumination light is sent to an illumination optical system of the front end portion 17 of the endoscope insertion portion 13. In addition, an electric connector is connected to the light guide connector. The electric connector is removably connected to a processor which performs image signal processing or the like.

The soft portion 19 is provided to extend from the consecutive joint portion 29 and have a narrower diameter than that of the consecutive joint portion 29. The soft portion 19 is formed out of a softer member than the rigidity of a member forming the consecutive joint portion 29.

The curved portion 21 is operated to be curved remotely by rotation of the angle knobs 23 and 25 of the body operation portion 11. Thus, the front end portion 17 is directed in a desired direction.

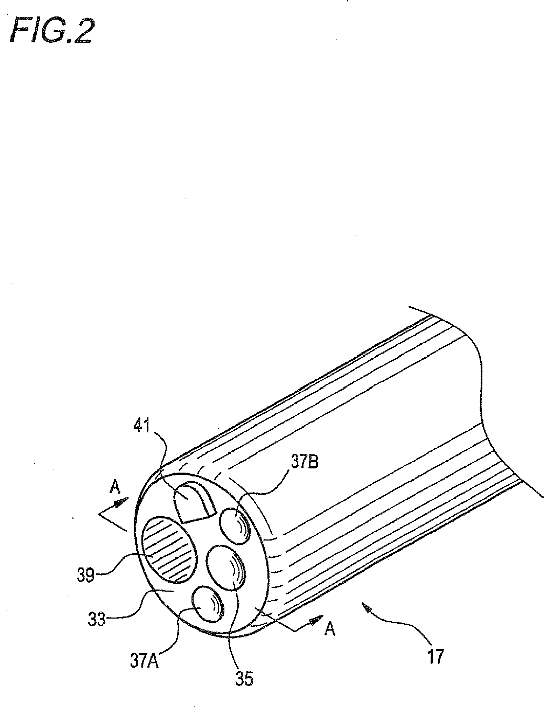

Fig. 2 is a schematic external view of the front end portion of the endoscope insertion portion.

In the front end portion 17 of the endoscope insertion portion 13, an observation window 35 is disposed in a front end surface 33, and illumination holes 37A and 37B of the illumination optical system, the forceps port 39 and an air/water-supply nozzle 41 are disposed on opposite sides of the observation window 35.

The observation window 35 imports light from a portion to be observed in a body cavity, and guides the light into an objective optical system in the front end portion 17 which will be described later.

The portion to be observed in the body cavity is irradiated with light from the illumination holes 37A and 37B.

The operative instrument such as forceps inserted from the forceps insertion portion 31 shown in Fig. 1 is led out from the forceps port 39.

The air/water-supply nozzle 41 supplies air/water toward the observation window 35.

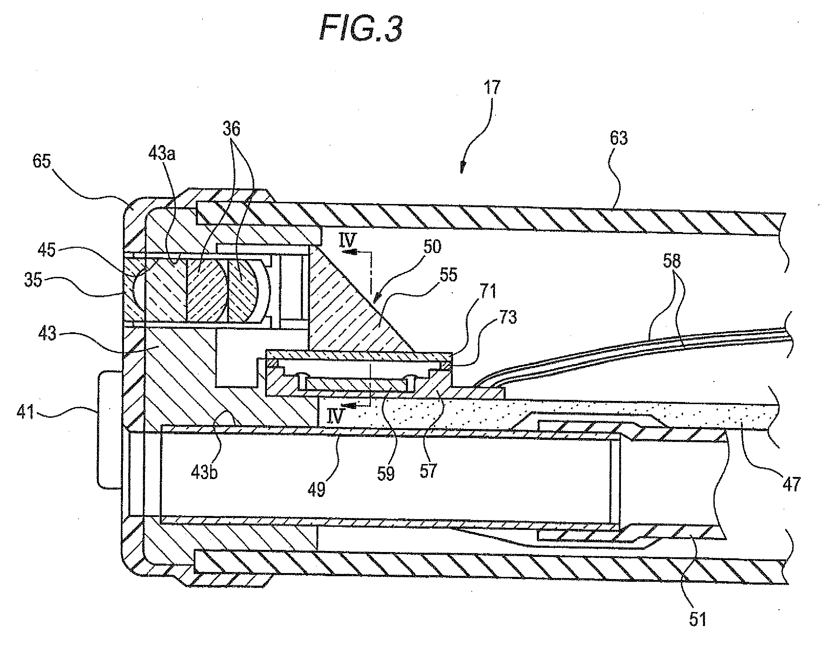

Fig. 3 is a sectional view taken along the direction of arrows A-A, showing the front end portion of the endoscope shown in Fig. 2.

The front end portion 17 has a front end hard portion 43 made from a metal material such as a stainless steel material, a casing tube 63 covering an outer circumference of the front end hard portion 43, and a front end cover 65 covering a front end side of the front end hard portion 43. The casing tube 63 and the front end cover 65 are bonded to each other tightly enough to prevent water from entering the inside of the front end portion 17.

A first bore hole 43a and a second bore hole 43b are formed in the front end hard portion 43. A lens barrel 45 is fitted and inserted into the first bore hole 43a. The lens barrel 45 receives the observation window 35 exposed in a front end surface of the front end portion 17, and an objective lens 36. A forceps pipe 49 made from metal is fitted and inserted into the second bore hole 43b, and a forceps tube 51 made from a soft material is connected to the forceps pipe 49. The observation window 35 and the objective lens 36 constitute the objective optical system.

In addition, an imaging unit 50 including the objective lens 36, a prism 55 and an image sensor 59 is built in the front end portion 17.

The prism 55 is disposed on an extension of the optical axis of the objective lens 36 retained in the lens barrel 45 in an internal space of the front end portion 17. The prism 55 is a triangular prism, by which light incident from the objective lens 36 side is reflected and guided onto an imaging surface of the image sensor 59.

The image sensor 59 is a CCD image sensor, which is mounted on a circuit board 57. A transparent cover glass 71 is bonded to the light entrance side of the circuit board 57 so as to seal the image sensor 59 air-tightly. The circuit board 57 is retained in the internal space of the front end portion 17 by a retention member 47. A signal cable 58 for outputting image information supplied from the image sensor 59 to a not-shown processor is connected to the circuit board 57.

The imaging unit 50 forms an image of the light imported through the objective lens 36, on the image sensor 59 mounted on the circuit board 57, through the prism 55. The imaging unit 50 outputs image information from the image sensor 59. The image information outputted from the imaging unit 50 is subjected to signal processing by the processor and displayed as an image on a monitor.

Though not shown, an illumination optical system including optical members such as lenses disposed in the illumination ports 37A and 37B (see Fig. 2), and a light guide, is disposed in the internal space of the front end portion 17 of the endoscope.

In the endoscope 100, the image sensor 59 or the circuit board 57 mounted with the image sensor 59 generates heat during observation with the endoscope. On the other hand, the temperature of the prism 55, the objective lens 36 or the observation window 35 is apt to be lower than that of the image sensor 59. Particularly water or air may be delivered toward the observation window when the observation window is smeared. In this case, the temperature of the prism 55, the objective lens 36 or the observation window 35 becomes lower than that of the image sensor 59. Therefore, a heat conduction portion for transmitting the heat of the image sensor 59 to the prism 55 is provided to suppress occurrence of a temperature difference between the inside and the outside of the cover glass.

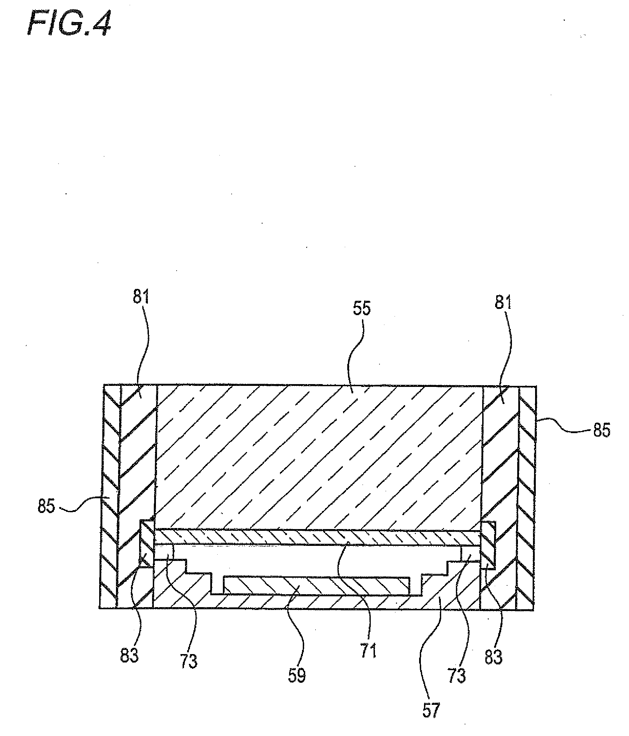

Fig. 4 is a sectional view taken along the direction of arrows IV-IV in Fig. 3.

The transparent cover glass 71 is bonded to the circuit board 57 mounted with the image sensor 59. The cover glass 71 seals the image sensor 59 air-tightly in a space between the cover glass 71 and the circuit board 57. The cover glass 71 is bonded to the circuit board 57 with interposition of a frame-like intervening portion 73 provided on an upper surface of the circuit board 57. However, the cover glass 71 may be bonded to the circuit board 57 directly without the intervening portion 73 provided therebetween. On the other hand, an upper surface of the cover glass 71 touches a lower surface of the prism 55.

Heat conduction portions 81 for transmitting heat of the image sensor 59 to the prism 55 are provided on opposite side surfaces of the circuit substrate 57 and the prism 55 so as to touch the prism 55 and extend consecutively from the prism 55 to the neighborhood of the image sensor 59.

Each heat conduction portion 81 is a film which contains a thermally conductive resin and has a predetermined thickness. The heat conduction portions 81 are in surface-contact with the outside side surfaces of the circuit board 57 and the side surfaces of the prism 55,

The thermally conductive resin includes at least one of an epoxy resin, an acrylic resin, a silicon resin and a urethane resin.

The thermally conductive resin may also include a thermally conductive filler. For example, ceramic (alumina, aluminum nitride, graphite, silicon carbide, etc.) may be used as the thermally conductive filler.

In addition, a heat insulation portion 83 is provided between each heat conduction portion 81 and the side surface of the cover glass 71 so as to avoid direct contact therebetween. In this example, the heat insulation portion 83 is provided to also cover the outside-facing side surface of the intervening portion 73. When the intervening portion 73 is formed out of a heat insulating material or a low thermally conductive material, the heat insulation portion 83 may be provided to cover at least the whole side surface of the cover glass 71.

An outside heat insulation portion 85 for preventing heat from being transmitted to members around the image sensor, which members are not shown in Fig. 4, is provided on the outside surface of each heat conduction portion 81. The same material as that for the heat insulation portion 83 may be used for the outside heat insulation portion 85.

According to the aforementioned endoscope. 100, when the imaging unit 50 is driven, the temperature in and around the image sensor 59 is higher than that of the objective lens 36 side due to heat generated by the driving. Then, the heat is transmitted from the image sensor 59 to the inner surface of the cover glass 71 on the image sensor 59 side, so as to increase the temperature in the inner surface of the cover glass 71. On the other hand, the heat generated in and around the image sensor 59 is transmitted to the prism 55 by the heat conduction portions 81. Thus, a temperature difference between the temperature of the prism 55 and the temperature of the image sensor 59 side is narrowed. As a result, occurrence of the temperature difference between the outer surface of the cover glass 71 on the prism 55 side and the inner surface of the cover glass 71 on the image sensor 59 side can be suppressed so that occurrence of dew condensation on the cover glass 71 is suppressed.

In addition, each heat insulation portion 83 is provided between each heat conduction portion 81 and the cover glass 71 in order to avoid direct contact between the heat conduction portion 81 and the cover glass 71. Thus, heat transmitted through the heat conduction portion 81 is prevented from being transmitted to the cover glass 71 so that the heat can be surely transmitted to the prism 55. In addition, when a heat insulation unit is configured to include not only the heat insulation portion 83 between each heat conduction portion 81 and the cover glass 71 but also the outside heat insulation portion 85 provided outside the heat conduction portion 81, heat transmitted through the heat conduction portion 81 is more surely prevented from being transmitted to any other portion than the prism 55.

The heat conduction portion may be changed appropriately if it can transmit the heat of the image sensor 59 to the prism 55. As a modification, the heat conduction portion may be a thermally conductive sheet. Any one of a silicon sheet, a carbon nano sheet and a graphite sheet may be used as the thermally conductive sheet. When the heat conduction portion is constituted by a thermally conductive sheet, the heat conduction portion can be formed uniformly with a small thickness while the thermal conductivity is secured. Thus, the heat conduction portion can be processed easily to be attached to the circuit board 57 and the prism 55.

In the aforementioned example, the heat conduction portion 81 is provided on each of the opposite side surfaces of the circuit board 57 and the prism 55 as shown by the sectional view of Fig. 4. However, the heat conduction portion 81 may be provided on either of the opposite side surfaces of the circuit board 57 and the prism 55. It is preferable that the heat conduction portion 81 is provided on each of the opposite side surfaces of the circuit board 57 and the prism 55 so that heat can be transmitted to the prism 55 uniformly and in a short time by the heat conduction portion 81.

In addition, the position where each heat conduction portion 81 touches the prism 55 is not limited to the side surface of the prism 55. The heat conduction portion 81 may be brought into surface contact with any surface of the prism 55 other than the light entrance surface and light exit surface of the prism 55 as long as the heat conduction portion 81 does not interfere with the optical path of incident light.

In the aforementioned configuration example, each heat conduction portion 81 is designed to be in contact with the circuit board 57. However, the heat conduction portion 81 may be in direct contact with the side surface of the image sensor 59.

## Claims

1. An imaging unit comprising:
an objective optical system that imports light of an image of a subject;
an image sensor that forms an image of the light and outputs an imaging signal;
a cover glass that is provided on an imaging surface of the image sensor and seals the image sensor air-tightly;
a prism that is disposed between the objective optical system and the cover glass so as to guide the light from the objective optical system to the imaging surface; and
a heat conduction portion that touches the prism and extends from the prism towards the image sensor so as to transmit heat of the image sensor to the prism.

2. The imaging unit according to claim 1 further comprising a circuit board which is mounted with the image sensor, wherein the heat conduction portion is disposed in contact with the circuit board.

3. The imaging unit according to claim 1, wherein the heat conduction portion is disposed in contact with the image sensor.

4. The imaging unit according to any one of claims 1 through 3, wherein the heat conduction portion is in surface-contact with a surface of the prism other than a light entrance surface and a light exit surface of the prism.

5. The imaging unit according to any one of claims 1 through 4, further comprising:
a heat insulation unit that is provided at least between the heat conduction portion and the cover glass so as to avoid direct contact therebetween.

6. The imaging unit according to claim 5, wherein:
the heat insulation unit is provided in an opposite surface of the heat conduction portion to a side where the heat conduction portion touches the prism.

7. The imaging unit according to any one of claims 1 through 6, wherein the heat conduction portion is formed out of a thermally conductive resin.

8. The imaging unit according to claim 7, wherein the thermally conductive resin includes at least one of an epoxy resin, an acrylic resin, a silicon resin and a urethane resin.

9. The imaging unit according to claim 7 or 8, wherein the thermally conductive resin includes a thermally conductive filler.

10. The imaging unit according to any one of claims 1 through 6, wherein the heat conduction portion is a thermally conductive sheet.

11. The imaging unit according to claim 10, wherein the thermally conductive sheet is any one of a silicon sheet, a carbon nano sheet and a graphite sheet.

12. An endoscope comprising:
an insertion portion in which an imaging unit according to any one of claims 1 through 11 is built.
